# EUROPEAN PATENT APPLICATION

(11) **EP 4 369 349 A1**
(43) Date of publication of application: **15.05.2024**
(21) Application number: 23208949.0
(22) Date of filing: 10.11.2023
(51) Int. Cl.: G16H 10/60, G16H 20/10, G16H 20/60, G16H 50/20

(54) **METHOD FOR PREDICTING ADVERSE SYMPTOMS TO IMMUNOTHERAPY**

(30) Priority: 11.11.2022 US 202263424579 P
(71) Applicant: Alerje, Inc., Detroit, MI 48202 (US)
(72) Inventor: EVELYN, Javier, Kalamazoo, 49009 (US)
(74) Representative: Kutzenberger Wolff & Partner

(57) **Abstract**

A method for predicting an adverse symptom to immunotherapy. The method comprises receiving a medical history and one or more therapy features, related to a food allergy, of a patient; evaluating a probability of the patient experiencing, respectively, each of one or more symptoms during immunotherapy; communicating the probability for each of the one or more symptoms to the patient and/or a physician of the patient.

## Description

### CROSS-REFERENCE TO RELATED APPLICATION

The present application claims priority to U.S. Provisional Application No. 63/424,579, filed on November 11, 2022, and incorporated herein by reference in its entirety for all purposes.

### FIELD

The present disclosure relates to a method for predicting adverse symptoms to immunotherapy. The method may employ machine learning for making said prediction, based on a training data set. The training set can be dynamically updated with genuine data and/or supplemented with synthetic data.

### BACKGROUND

Immunotherapy is a medical treatment used to aid patients in overcoming allergies by exposure to allergens. Since immunotherapy involves exposure of small, predetermined quantities of pharmaceutical-grade allergens by patients, it is expected that at some point during therapy the patient could present adverse symptoms. Moreover, patients may, through their diet, accidentally ingest allergens during immunotherapy (e.g., eating a dish that is cross-contaminated), increasing the risk of adverse symptoms.

There is a great deal of anxiety associated with having a food allergy and this anxiety can be even more acute for patients undergoing immunotherapy in which the probability of presenting adverse symptoms is increased by regular exposure. Some of this anxiety may be attributed to not being certain which adverse symptoms will be presented, and whether the adverse symptoms will be minor (e.g., requiring merely an oral dose of antihistamine) or serious (e.g., requiring a trip to the Emergency Room).

Anxiety may not only be felt by the patient but also those around the patient like family and friends. Parents of children undergoing immunotherapy may oversee the therapy and monitor the children during dosing regimens. Since young children (e.g., ages 10 and under) typically do not communicate well or fully understand what is happening to their bodies during an adverse reaction, it is important for a parent to know what symptoms to look out for so action, if needed, can be taken quickly.

The particular symptoms felt are important to determine what action, if any, should be taken. Minor reactions, while monitoring is recommended, may not need immediate drastic action such as administering epinephrine or a trip to the Emergency Room. Knowing what symptoms to expect and what to do when they are exhibited by the patient may save the patient and/or the guardian thereof time and expense. Moreover, such knowledge can alleviate overburdening of the medical system by occupying physician time with patient visits that are cautious but not necessary.

Food allergies afflict approximately 32 million people in the United States and approximately 200,000 people require emergency medical care every year due to their food allergies, according to FARE ("Food Allergy Research & Education"). Those afflicted suffer from reduced quality of life, as described above. While immunotherapy is currently available on the market today, there remains a need to increase participation and compliance by patients, as well as facilitating physicians' service to their patients.

There is a need for a method and system to predict adverse symptoms associated with allergies.

There is a need for a method and system to inform patients and/or their guardians of expected symptoms to prepare them for action, if needed.

There is a need for a method and system that dynamically updates training data sets for continuous prediction accuracy improvements.

There is a need for a method and system that can supplement a sparse data set with synthetic data to contribute to prediction accuracy.

### SUMMARY

The present disclosure relates to a method which may address at least some of the needs identified above. The method may comprise receiving a medical history and one or more therapy features, related to a food allergy, of a patient. The method may comprise evaluating a probability of the patient experiencing, respectively, each of one or more symptoms during immunotherapy. The method may comprise communicating the probability for each of the one or more symptoms to the patient and/or a physician of the patient.

The medical history may include gender, one or more allergy foods, one or more allergy severities associated with the one or more allergy foods, one or more known prior reactions including a quantity and/or one or more symptom identifications, skin prick test results, allergen specific Immunoglobulin E antibody test results, allergen specific Immunoglobulin E antibody serum test results, vitamin D level, history of asthma, history of eczema, history of anaphylaxis, history of environmental allergies, history of environmental sublingual immunotherapy prior to oral immunotherapy, start and/or end date of the environmental sublingual immunotherapy, history of environmental subcutaneous immunotherapy prior to oral immunotherapy, start and/or end date of the environmental subcutaneous immunotherapy, epinephrine autoinjector use history during the environmental sublingual immunotherapy, epinephrine autoinjector use history during the environmental subcutaneous immunotherapy, or any combination thereof. Preferably the medical history may include at least allergen specific Immunoglobulin E antibody serum test results.

The one or more therapy features may include age at the start of immunotherapy, food challenge history prior to oral immunotherapy, food sublingual immunotherapy history prior to oral immunotherapy, whether the oral immunotherapy involves treatment for a single or multiple food allergies, or any combination thereof. Preferably the one or more therapy features may include at least age at the start of immunotherapy and whether the oral immunotherapy involves treatment for a single or multiple food allergies.

The immunotherapy may be oral immunotherapy in which the patient orally ingests a medication comprising a component of an allergen food on a predetermined schedule, including a plurality of maintenance phases in which a dosage remains constant and a plurality of up-dosing phases in which the dosage is increased relative to an immediately prior maintenance phase.

The one or more symptoms may include anaphylaxis, cutaneous symptoms, respiratory symptoms, abdominal pain and/or nausea without vomiting, nausea with vomiting, development of eosinophilic esophagitis, or any combination thereof.

The receiving and evaluating steps may be performed by a machine learning model. The machine learning model may include a light gradient-boosting machine framework. The machine learning model may include a logistic regression algorithm.

The machine learning model may be trained by supervised learning.

The machine learning model may be trained with one or more training sets of data comprising genuine patient data, synthetic data, or both. The genuine patient data may be anonymized.

The one or more training sets of data may undergo one or more transformations including replacing missing values, encoding categorical data into numerical data, standardizing data scales, balancing, or any combination thereof. The balancing may exclude at least some of the one or more training sets of data such that a ratio of data sets where no symptoms are experienced during therapy to data sets where symptoms are experienced during therapy is about 70:30 or less, more preferably about 65:35 or less, more preferably 60:40 or less, more preferably about 55:45 or less, or even more preferably about 50:50.

The present disclosure relates to a non-transient memory storage medium comprising computer executable instructions for performing the method according to one or any combination of the paragraphs above.

The non-transient memory storage medium may be local to a computing device of the patient or local to a computing device of the physician. The computer executable instructions may be carried out by one or more processors local to a computing device of the patient or local to a computing device of the physician.

The present disclosure relates to a method which may address at least some of the needs identified above. The method may be employed for predicting an adverse symptom to immunotherapy. The method may comprise training a machine learning model with a training set of medical history, pre-OIT data, and peri-OIT data. The method may comprise obtaining a patient set of medical history, pre-OIT data, and peri-OIT data. The method may comprise predicting a) if the adverse symptom will present in a patient, and b) a type of the adverse symptom that will present in the patient.

The adverse symptom may include, but is not limited to, anaphylaxis, cutaneous symptoms (e.g., hives, rashes, and/or eczema), abdominal pain, nausea, respiratory symptoms (e.g., wheezing, nasal congestion, and/or trouble breathing), eosinophilic esophagitis ("EOE"), swelling (e.g., of the lips, face, tongue, and/or throat), cardiac symptoms (e.g., dizziness, lightheadedness, fainting, and/or rapid pulse), the like, or any combination thereof.

The patient may be undergoing oral immunotherapy (OIT).

The medical history may include, but is not limited to, gender, most recent IgE value prior to starting OIT, skin prick test results (i.e., wheal diameter typically measured in mm), allergen specific serum test results (e.g., measured in IgE kU/L) that tests for allergies to specific substances, vitamin D level prior to OIT, history of other diseases (e.g., asthma, eczema, anaphylaxis, and the like), allergens (e.g., food, environmental, and/or chemical), whether the patient has multiple food allergies, or any combination thereof; wherein the history of other diseases and/or the allergens may indicate if the adverse symptom could be attributed to something other than OIT.

The pre-OIT data may include age at start of OIT, food challenge for OIT-targeted food in which in a hospital setting the patient is given increasing quantity of the OIT-targeted food with IgE values being measured during the same, whether food sublingual immune therapy prior to OIT was performed, whether multiple food allergies are being treated in one OIT round, or any combination thereof.

The peri-OIT data may include the presentation during therapy of: anaphylaxis, cutaneous symptoms, abdominal pain and/or nausea without vomiting, respiratory symptoms, nausea with vomiting, EOE, cardiac symptoms, or any combination thereof.

The medical history and/or pre-OIT data may be obtained from medical records via an application programming interface (API) or provided as an input by the patient and/or a caregiver of the patient.

The peri-OIT data may be obtained via an application.

The application may be stored on and/or executed by a computing device (e.g., a smart phone).

The training data may be dynamically updated with data obtained via the application.

The training data may be supplemented with synthetic data.

The machine learning model may employs a light gradient-boosting machine framework in a machine learning model.

The synthetic data may be obtained by employing a light gradient-boosting machine framework in a machine learning model.

The present disclosure relates to a system which may address at least some of the needs identified above. The system may be employed for performing the method of the present disclosure. The system may comprise a computing device. The computing device may comprise a non-transitory memory storage medium storing computer-executable instructions for performing the method; and a processor for executing the computer-executable instructions.

The system may further comprise a second computing device from which the medical history and/or pre-OIT data is obtained. The computing device and the second computing device may communicate via a network. The computing device may retrieve the medical history and/or pre-OIT data from the second computing device.

### BRIEF DESCRIPTIONS OF THE DRAWINGS

**FIG. 1** illustrates a graph corresponding to Example 1.
**FIG. 2** illustrates a graph corresponding to Example 1.
**FIG. 3A** illustrates a graph corresponding to Example 1.
**FIG. 3B** illustrates a graph corresponding to Example 1.
**FIG. 3C** illustrates a graph corresponding to Example 1.
**FIG. 3D** illustrates a graph corresponding to Example 1.
**FIG. 3E** illustrates a graph corresponding to Example 1.
**FIG. 3F** illustrates a graph corresponding to Example 1.
**FIG. 4A** illustrates a graph corresponding to Example 1.
**FIG. 4B** illustrates a graph corresponding to Example 1.
**FIG. 4C** illustrates a graph corresponding to Example 1.
**FIG. 4D** illustrates a graph corresponding to Example 1.
**FIG. 4E** illustrates a graph corresponding to Example 1.
**FIG. 4F** illustrates a graph corresponding to Example 1.
**FIG. 5A** illustrates a graph corresponding to Example 1.
**FIG. 5B** illustrates a graph corresponding to Example 1.
**FIG. 5C** illustrates a graph corresponding to Example 1.
**FIG. 5D** illustrates a graph corresponding to Example 1.
**FIG. 5E** illustrates a graph corresponding to Example 1.
**FIG. 5F** illustrates a graph corresponding to Example 1.
**FIG. 6** illustrates a graph corresponding to Example 2.

### DETAILED DESCRIPTION

The present teachings relate to a machine learning model ("ML model" or "model") and a method of predicting adverse symptoms using the machine learning model.

The present teachings address gaps in immunotherapy that have hindered the widespread adoption of immunotherapy. In addition to hesitation by patients to commit to immunotherapy, physicians have been limited in the tools for managing higher caseload volumes, accurately informing patients of risks, and obtaining comprehensive data and trends on populations of patients with food allergies.

The ML model discussed herein may be trained with genuine and/or synthetic data and validated to operate with a high degree of accuracy. Genuine data may refer herein to data of a real patient, such as existing in medical records. Synthetic data may refer herein to data generated by the ML model based on training with at least some genuine data.

In terms of accuracy, it is understood that accuracy generally increases with an increased volume of training data. In this regard, population sizes of about 200 or more, more preferably about 400 or more, more preferably about 600 or more, more preferably about 800 or more, or even more preferably about 1,000 or more may be included in the training data. This quantity may increase as immunotherapy adoption increases within the US and across the globe (e.g., to the tens or even hundreds of thousands). This quantity may increase with the introduction of synthetic data as described herein (e.g., to the tens or even hundreds of thousands). Thus, heretofore, there has existed a gap in knowledge that is addressed by the present teachings. It is understood that data sets processed by the ML model described herein also include a plurality of prediction parameters for each patient in the population.

Many challenges hinder conventional technologies and methods from performing the predictions discussed herein. Physicians conventionally do not have access to comprehensive records of patients who are not under their care. Many records are incomplete as to at least some relevant data (e.g., some of the prediction parameters discussed herein). There are gaps in studies due to food allergy immunotherapies being relatively new and having a limited number of patients participating. Even if studies are performed, typically they can be limited in scope. Costs and efforts of performing these studies hinder population size volume and study regularity - one challenge of which is obtaining access to, analyzing, or anonymizing data, or any combination thereof.

With respect to anonymizing data, it is understood that medical records are subj ect to various rules, laws, and regulations preventing access, at least without consent of the patient. Heretofore, there have not been comprehensive studies providing the correlations of prediction parameters with symptoms triggered by immunotherapy as described herein.

In some aspects, the symptom information may guide a patient's decision whether or not to start treatment. Treatment may take place during a period of months or even years. In this regard, a patient may evaluate the type, probability, and severity of symptoms and decide whether they would prefer to start treatment at present, in the future, or even at all. Some anticipated life events may also be accounted for in the ultimate decision of the patient.

In regard to the above, at least some of the training data population may be collected by a journalling application for managing immunotherapy. An exemplary application is described in U.S. Application No. 17/377,063, incorporated herein by reference in its entirety for all purposes. Such applications may provide a tool for patients to journal about their lifestyle, diet, and/or medication related to immunotherapy, leveraging smartphone technology. Thus, a pool of data may be provided for training the ML model and/or an individual patient's input into the ML model for symptom prediction may be facilitated. At least one benefit to such applications may be with the anonymization of data prior to extraction of data for use as a training data set. In this regard, data may be anonymized, removed from the data set, encrypted, or any combination thereof prior to any network communication of the data by the device of the patient.

Another aspect of anonymity is realized by the synthetic data described herein. That is, based on a finite set of genuine data used to train the ML model, synthetic data may be generated and fed back into the ML model for training, validating, and testing. Synthetic data, not being associated with a real patient, may be beneficial to overcoming the limitations to medical records access.

Synthetic data and/or the missing value replacement described herein may be beneficial to gaps in data sets. Understandably, medical histories may not be fully populated with all of the prediction parameters described herein. This tends to be more common with laboratory tests. It has been observed that parameters that are most commonly missing include vitamin D level, skin prick test results, allergen specific Immunoglobulin E antibody test results, allergen specific Immunoglobulin E antibody serum test results, and known prior reactions.

The functioning of the ML model and subsequent accuracy testing may be improved by the learning described herein, as well as the predictions ultimately generated by the ML model. That is, the speed and/or accuracy of the ML model may improve over time based on the training and prediction making. The ML model described herein may develop correlations between prediction parameters and symptoms, thereby improving the ML model by applying greater weights to stronger correlations and diminishing the weights for weaker correlations. Such correlations may be developed as the ML model continues to operate.

Another benefit of the present teachings is the increased knowledge of patients and facilitation of doctor-patient interactions. By the symptom prediction described herein, patients may not be surprised by the occurrence of a predicted symptom and physicians may inform patients on the measures to treat the symptoms. In this regard, the quality of life of patients may be increased while participation and compliance with immunotherapies may be increased. In another aspect, burdens on the medical system may be diminished by reducing the incidence of unnecessary emergency medical facility visits where symptoms are predicted and patients can be aware of appropriate treatment methods.

Some aspects of the present teachings may be described from the perspective of oral immunotherapy ("OIT"), but it is contemplated that the ML model and method described herein may be used for other forms of immunotherapy such as sublingual immunotherapy ("SLIT") and subcutaneous immunotherapy ("SCTT").

At least some of the more prevalent food allergies addressed by the present teachings may include hazelnut, sesame, walnut, cashew, milk, egg, peanut, coconut, sunflower, pistachio, macadamia, almond, lentil, pecan, wheat, rye, barley, chicken, chickpea, green pea, tomato, soy, flaxseed, or any combination thereof. Although, the present teachings may also address one or any combination of the more than 170 currently identified food allergies.

The present teachings may refer to a maintenance phase and an up-dose phase. As immunotherapy progresses, typically one aim is to progressively increase the dosage of medication (e.g., Palforzia being one exemplary medication adapted for immunotherapy adapted for peanut allergies). Maintenance phases may refer to a point or period of time in which the patient takes a consistent dosage of medication. Up-dose phases may refer to a point or period of time in which the patient takes an increased dosage of medication, relative to the preceding maintenance phase. In some circumstances up-dose phases may involve a patient taking a progressively increased dosage of medication during a single up-dose phase until a predetermined dose is achieved. It is possible that due to the comfort of the patient, any reactions, medical emergencies, or the like, physicians may recommend that dosage be reduced for a time, at least until another up-dose phase is attempted. It is also possible that physicians can recommend to cease immunotherapy altogether.

The ML model may be trained with one or more training sets of data. The training sets of data may be drawn from one or more populations. The one or more populations may comprise a plurality of patients. One or more of the prediction parameters described herein may be provided in the one or more training sets of data. For each patient, symptom data may be included to train the model with supervised learning.

Training sets of data may be obtained via journalling inputs from the patients, inputs from physicians, medical records, insurance records, the like, or any combination thereof. As described herein, synthetic data generated through the ML model may be employed for training. Training data sets may be anonymized such that individual patients may not be identifiable through the data sets. This may include omitting or obfuscating names, birth dates, addresses, phone numbers, the like, or any combination thereof. In some aspects, the present teachings may not require any information for model training, that can be traced back to an individual.

The training sets of data may be structured as a table with rows corresponding to individual patients and columns corresponding to prediction parameters. The tabular format may be understood as a data format which the ML model is adapted to operate, although any other suitable data format may be within the scope of the present teachings.

Training data may be prepared for training the ML model by one or more transformations. The one or more transformations may include replacing missing values, encoding, standardization, or any combination thereof.

Replacing missing values may be based on an average or other basis (e.g., min or max). The average or other basis may operate individually for each prediction parameter (e.g., a column arranged in a table). Missing values may be present due to lack of inputs. For instance, an allergen specific serum may not have been recorded for a patient, or a patient may accidentally omit a journalling input. Replacing missing values may be advantageous in the present teachings as knowledge gaps in the prediction parameters described herein are not uncommon.

Encoding may convert categorical data into numerical data. That is, data typically identified with a text string (e.g., male, female, peanut allergy, etc.) may be converted into numerical data. An exemplary encoding method may include one-hot encoding.

Standardization may account for differences in scales across different prediction parameters. For instance, allergen specific serum is measured quantitatively in IgE kU/L, whereas age at the start of therapy is measured quantitatively in years. Without standardization, biases may arise in the machine learning. An exemplary standardization method may include adjusting the data distribution (within a single column, and performed separately for each column) to have a mean of 0 and a standard deviation of 1.

One or more of the transformations described above may be arranged in a pipeline. That is, the transformations may be sequenced. The sequence may proceed as: replacing missing values, encoding, and standardization.

One or more of the transformations described above may be selectively performed on one or more columns. That is, some transformations may be performed on a first set of columns but not another set of columns. For example, encoding may not need to be performed on allergen specific serum data, which is inherently a numerical value.

Training data may be split into a training set, a validation set, and a test set. Such splitting may ensure that data sets for a patient aren't duplicated between the training, validation, and testing. In this regard, bias may be eliminated from testing, whereby data used for training and/or validating is not used for testing final model performance.

Estimations of the ML model may be tested based on a framework of true positive, true negative, false positive, false negative. At least some of the training data may comprise information to determine whether a positive or negative is true or false.

In this regard, one or more evaluations may be performed upon the outputs of the testing. The evaluations may include an F1 score, a confusion matrix, a classification report, an AUC-ROC curve, support vector machines, or any combination thereof.

The F1 score may characterize the harmonic mean of precision (correct positive predictions relative to total positive predictions) and recall (correct positive predictions relative to total actual positives), which are measures different from accuracy (percentage of all correctly classified observations).

Support vector machines may separate data points using a hyperplane with the largest amount of margin between different classes. Thus, new data points plotted with existing data points may be classified in accordance with their plot coordinates with respect to the hyperplane.

Based on the foregoing, one or more weights or coefficients of the algorithm employed by the ML model may be adjusted to improve the F1 score, which is about 0.6 or more, more preferably about 0.7 or more, more preferably about 0.8 or more, or even more preferably about 0.9 or more. The weights or coefficients may indicate the strength of correlations between one or more prediction parameters and one or more symptoms. In some aspects, correlations may be made between individual prediction parameters and individual symptoms. In some aspects, correlations may be made between multiple prediction parameters and individual symptoms, or vice versa. In some aspects, some prediction parameters may strengthen or diminish the correlations of one or more other prediction parameters (e.g., as described herein, some symptoms are age-dependent).

The population dealt with herein may be unbalanced between those that experience no symptoms during therapy and those that experience symptoms during therapy, which can negatively impact model accuracy. Typically, more individuals will experience symptoms than those who don't experience symptoms. In this regard, the data may be filtered such that the balance of the latter (symptoms) to the former (no symptoms) is about 70:30 or less, more preferably about 65:35 or less, more preferably 60:40 or less, more preferably about 55:45 or less, or even more preferably about 50:50. In this regard, the total data set may be reduced by removing the class with more individuals to better balance the data. In the interest of avoiding the reduction of the total data set, it has been found that a balance of about 65:35 may be suitable for model training.

The model may function to predict the probability that a set of prediction parameters for a patient belong in a given class. The classes may be defined as, *inter alia*, likely to have an adverse reaction during therapy, likely not to have an adverse reaction during therapy, likely to exhibit anaphylaxis during therapy, likely to exhibit abdominal pain during therapy, likely to exhibit cutaneous symptoms during therapy, likely to exhibit respiratory symptoms during therapy, likely to exhibit nausea with vomiting during therapy, likely to exhibit abdominal pain and/or nausea without vomiting during therapy, likely to exhibit eosinophilic esophagitis ("EoE"), or any combination thereof.

The foregoing is merely exemplary and the present teachings contemplate that the ML model may predict the likelihood of any other symptoms described herein and known in the art as associated with a food allergy.

The ML model may receive inputs of one or more prediction parameters (preferably two, three, four, five, or even six or more prediction parameters) and provide one or more outputs, estimating a class based on the inputs. The estimation may be based on the training of the ML model described above.

The model may use a light gradient-boosting machine framework. This framework may be based on weak learner decision trees where each learner improves the model of previous learner by decreasing previous misclassified data. The light gradient-boosting machine framework may leverage both gradient-based one side sampling and exclusive feature bundling techniques.

The model described herein may include a logistic regression algorithm. The logistic regression algorithm finds suitability in the present teachings based on the binary decision-making that is involved. That is, typically the prediction being made is whether or not any symptoms or one or more specific symptoms will be exhibited by the patient during therapy.

The model described herein may receive prediction parameters as inputs and provide a probability as an output. Probably may refer to whether or not a patient will exhibit symptoms during immunotherapy, which specific symptoms described herein will occur, or both.

The prediction parameters may be classified as historical features, therapy features, and symptom features.

Historical features may include the medical history of the patient. The historical features may include gender, one or more allergy foods, one or more allergy severities associated with the one or more allergy foods, one or more known prior reactions including a quantity and/or one or more symptom identifications, skin prick test results, allergen specific Immunoglobulin E antibody test results, allergen specific Immunoglobulin E antibody serum test results, vitamin D level, history of asthma, history of eczema, history of anaphylaxis, history of environmental allergies, history of environmental sublingual immunotherapy prior to oral immunotherapy, start and/or end date of the environmental sublingual immunotherapy, history of environmental subcutaneous immunotherapy prior to oral immunotherapy, start and/or end date of the environmental subcutaneous immunotherapy, epinephrine autoinjector use history during the environmental sublingual immunotherapy, epinephrine autoinjector use history during the environmental subcutaneous immunotherapy, or any combination thereof.

It has been found that allergen specific Immunoglobulin E antibody serum test results provides strong correlations to symptoms and thus the ML model may give a greater weight to this prediction parameter.

Therapy features may include information regarding immunotherapy, including prior immunotherapies and a current round of immunotherapy. The therapy features may include age at the start of immunotherapy, food challenge history prior to oral immunotherapy, food sublingual immunotherapy history prior to oral immunotherapy, whether the oral immunotherapy involves treatment for a single or multiple food allergies, or any combination thereof.

Prior immunotherapy history may include whether the patient graduated from the immunotherapy, immunotherapy was discontinued prior to graduation, whether the patient was transitioned to a different form of immunotherapy (e.g., OIT, SLIT, SCIT), or any combination thereof. Prior epinephrine history may include whether an epinephrine autoinjector was used during a maintenance phase or an up-dose phase, and where it was used (e.g., home, office, etc.). Anaphylaxis history may include whether the patient exhibited anaphylaxis during a maintenance phase or an up-dose phase.

It has been found that age at the start of immunotherapy and whether the oral immunotherapy involves treatment for a single or multiple food allergies provides strong correlations to symptoms and thus the ML model may give a greater weight to this prediction parameter.

Symptom features may include information regarding symptoms predicted to be exhibited by the patient during immunotherapy. The symptom features may include anaphylaxis, cutaneous symptoms, respiratory symptoms, abdominal pain and/or nausea without vomiting, nausea with vomiting, development of eosinophilic esophagitis, or any combination thereof. The model described herein may be trained to correlate one or more prediction parameters with symptom features.

The foregoing is exemplary and it is understood that any other prediction parameters discussed herein is incorporated into the paragraphs above.

### Example 1.

Prediction parameters discussed herein have been selected based on testing the relationships between a patient's historical features and/or therapy features , and symptom features. The goal was to determine what symptoms had a link to one or more of the medical history parameters. In this manner, one or more adverse symptoms in individual patients - both whether the symptoms will arise and the type of symptoms that will arise - can be accurately predicted.

A study was conducted on a population of patients suffering from food allergies and undergoing OIT. Feature variables were obtained for each patient. During the study, symptoms were tracked through a digital application accessible by any conventional smartphone. Symptoms, if they occurred, were characterized by the date and time of occurrence, the type of the symptom, and the severity of the symptom. Also, other data can be collected such as the duration of the symptoms, action taken to address the symptoms (e.g., taking an oral dose of anti-histamine, dosing with epinephrine, an Emergency Room visit, or the like), whether no action was taken (e.g., the patient was merely monitored until the symptoms subsided), the patient's anxiety level due to the adverse symptoms, the like, or any combination thereof.

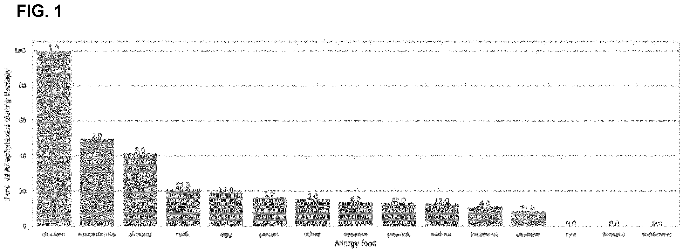

It was found that there was generally not a strong relationship between the type of allergen and the occurrence of a symptom. **FIG. 1** illustrates the percentage of adverse symptoms encountered during OIT presenting as anaphylaxis for individual allergens, on a y-axis of 1 to 100%. From left to right, the allergen and associated sample size includes: chicken/1.0, macadamia/2.0, almond/5.0, milk/17.0, egg/17.0, pecan/1.0, other/2.0, sesame/6.0, peanut/42.0, walnut/12.0, hazelnut/4.0, cashew/11.0, rye/0.0, tomato/0.0, and sunflower/0.0. Allergies to chicken, macadamia nuts, pecans, etc. are generally not common and therefore the sample sizes are understandably limited. These limitations may be realized in at least some other data sets discussed herein. However, strong data was obtained for the allergens milk, eggs, sesame, peanut, and cashews.

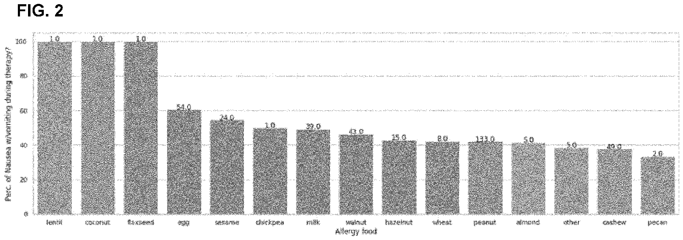

It was found that some allergens are more likely to cause certain symptoms. For example, both eggs and sesame are more likely to cause nausea with vomiting. **FIG. 2** illustrates the percentage of adverse symptoms encountered during OIT presenting as nausea with vomiting for individual allergens, on a y-axis of 1 to 100%. From left to right, the allergen and associated sample size includes: lentil/1.0, coconut/1.0, flaxseed/1.0, egg/54.0, sesame/24.0, chickpea/1.0, milk/39.0, walnut/43.0, hazelnut/15.0, wheat/8.0, peanut/133.0, almond/5.0, other/5.0, cashew/49.0, and pecan/2.0.

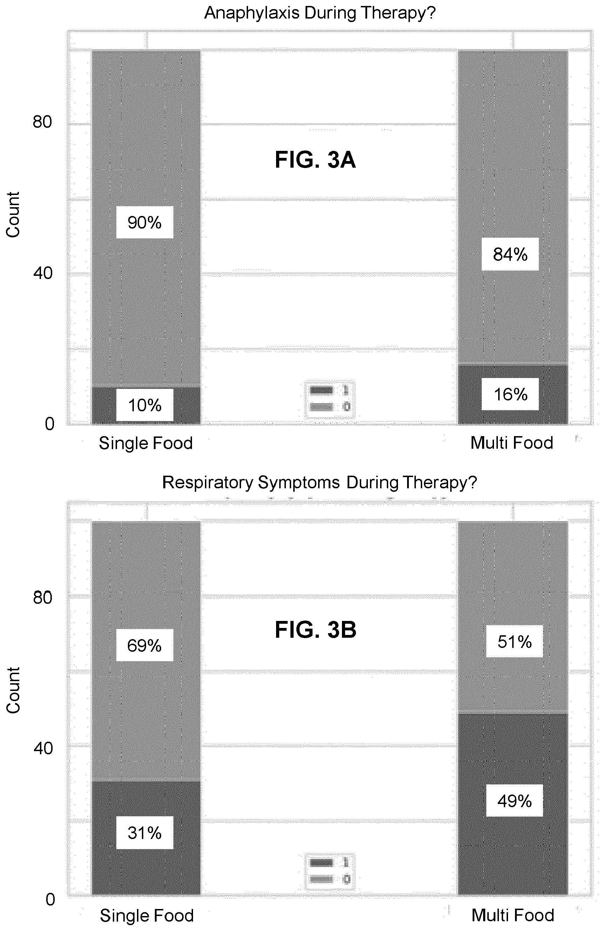

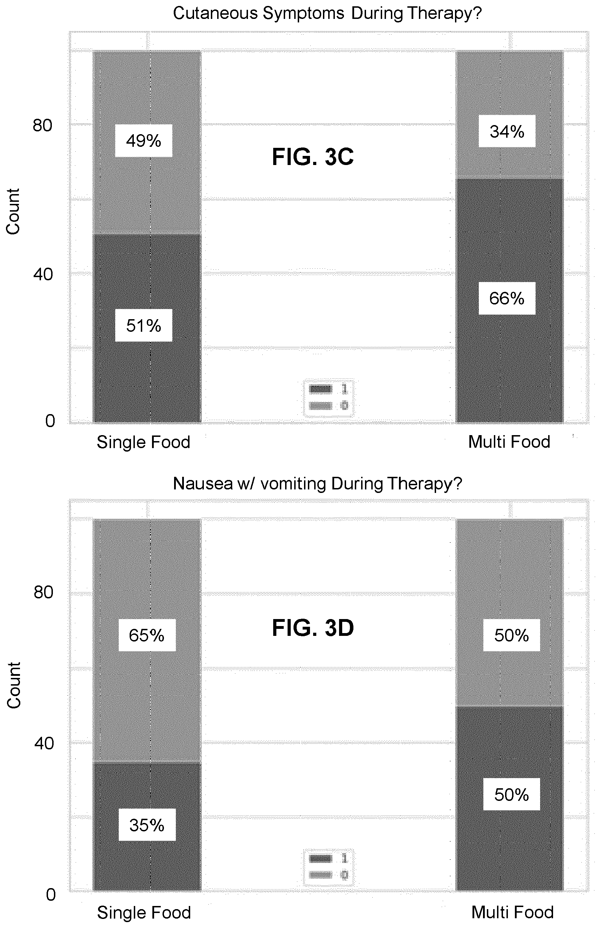

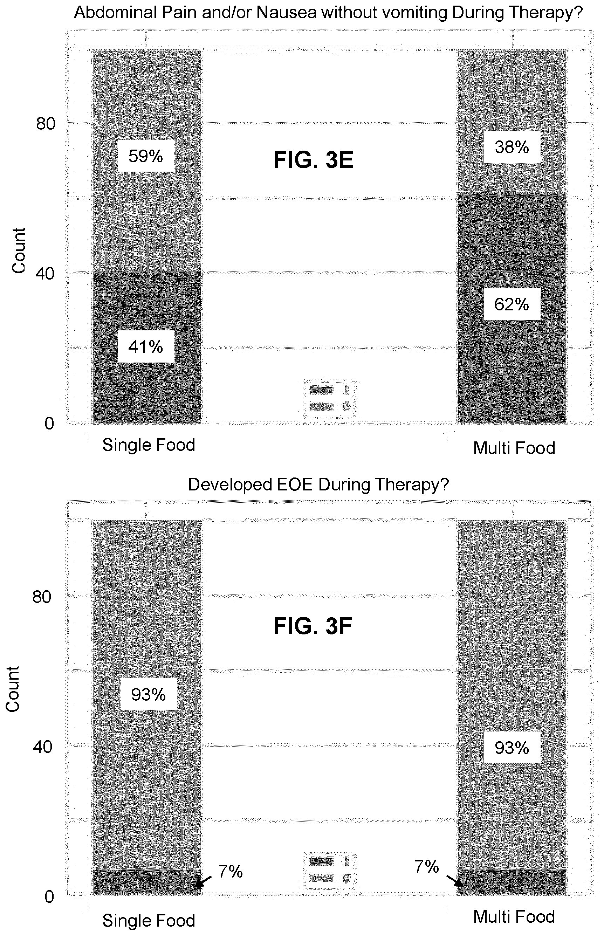

It was found that patients undergoing OIT for multiple allergies were more likely to present symptoms relative to patients undergoing OIT for a single allergy. Except this trend was not definitive for eosinophilic esophagitis. **FIGS. 3A-3F** illustrates a series of charts, each of which are directed to a different symptom. The left bar is indicative of patients undergoing treatment for a single allergen and the right bar is indicative of patients undergoing treatment for multiple allergens. The percentage of patients not developing symptoms is indicated in the top portion of the bar while the percentage of patients developing symptoms is indicated in the lower portion of the bar.

It was found that as allergen specific serum increases, patients were more likely to present symptoms. Except this trend was not definitive for eosinophilic esophagitis. Allergen specific serum characterizes the quantity of Immunoglobulin E from a blood test, measured in IgE kU/L. To tailor this quantity more easily to the purposes of the present teachings, the quantity was broken down into 7 different categories, as shown below, although other category breakdowns are contemplated by the present teachings.

| Allergen specific serum | |
|---|---|
| Range of test result | Category |
| < 0.35 | Unlikely |
| 0.35 < result < 0.69 | Doubtful |
| 0.7 < result < 3.49 | Small possibility |
| 3.5 < result < 17.49 | Greater possibility |
| 17.5 < result < 49.99 | Very likely |
| 50 < result < 100 | Very high likelihood |
| Result = 100 | Extremely likely |

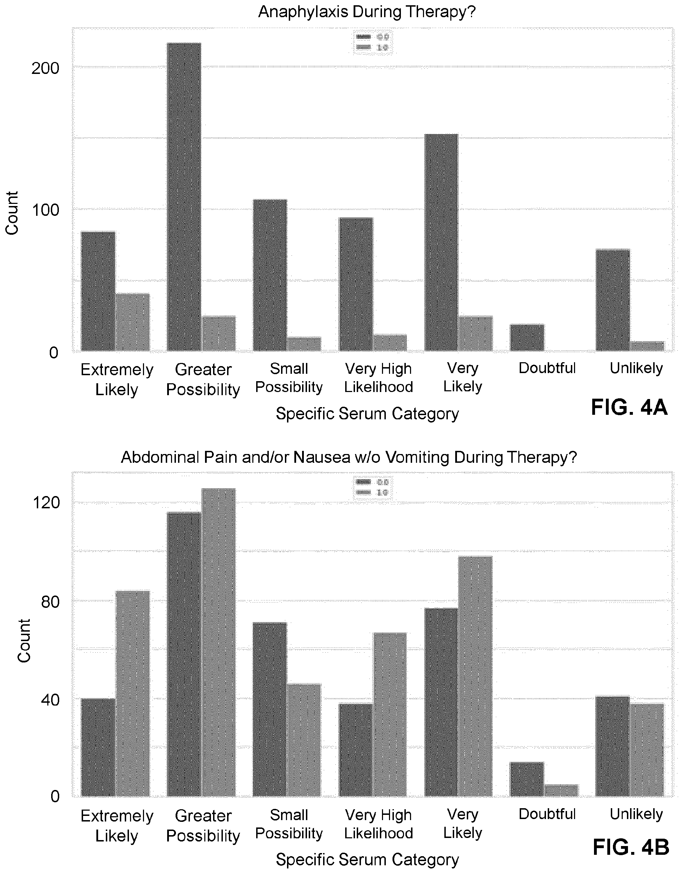

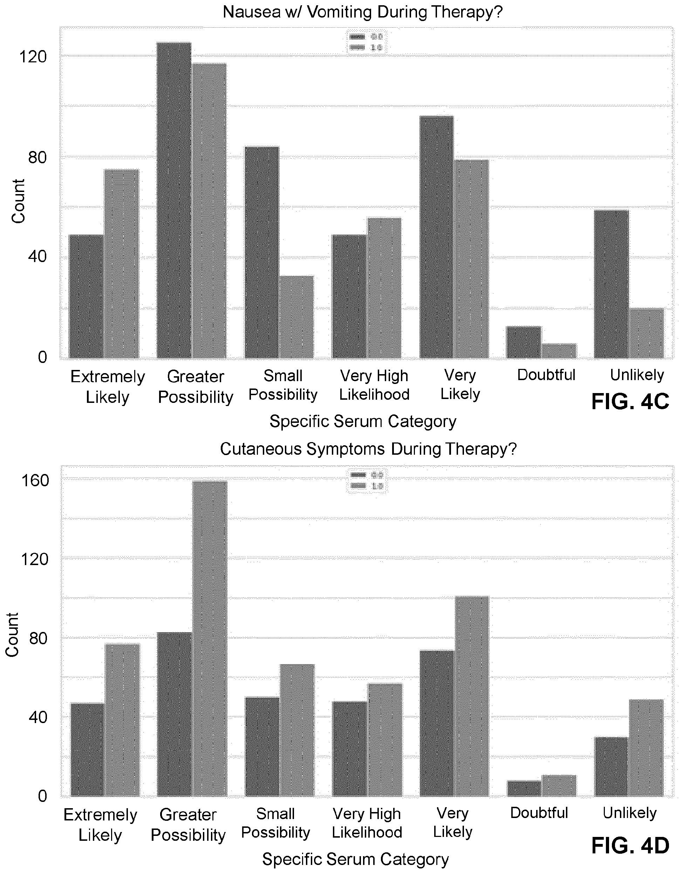

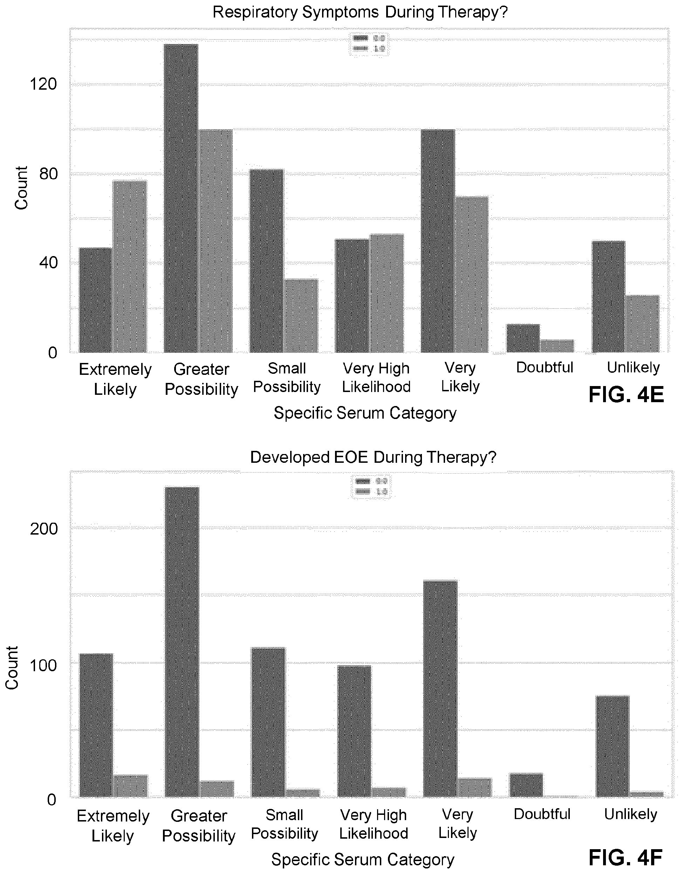

**FIG. 4A-4F** illustrates a series of charts, each of which are directed to different symptoms. The count (y-axis) of patients in each of the allergen specific Immunoglobulin E serum categories (x-axis) experiencing or not experiencing the symptom is presented. Those who did not experience symptoms is represented by the bar to the left in each cluster. Those who did experience symptoms is represented by the bar to the right in each cluster.

It was found that certain age groups are more likely to present different symptoms. For example, early ages are more likely to present cutaneous anomalies and nausea, relative to older age groups, while as age increases, patients are more likely to present abdominal pain, relative to early ages. To tailor this quantity more easily to the purposes of the present teachings, age was broken down into the following groups: 0-5, 5-10, 10-18, and 18+, although other age range breakdowns are contemplated by the present teachings.

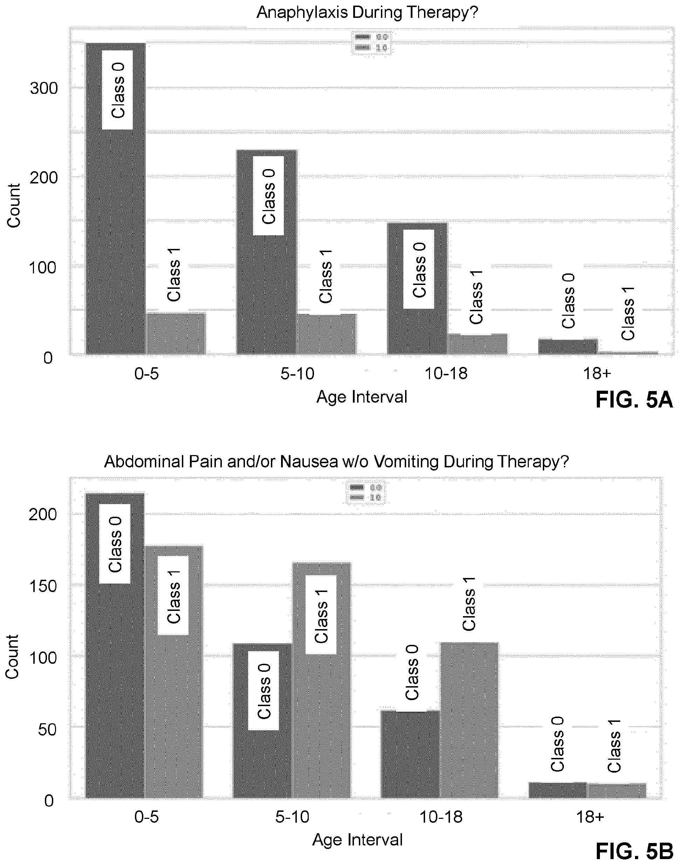

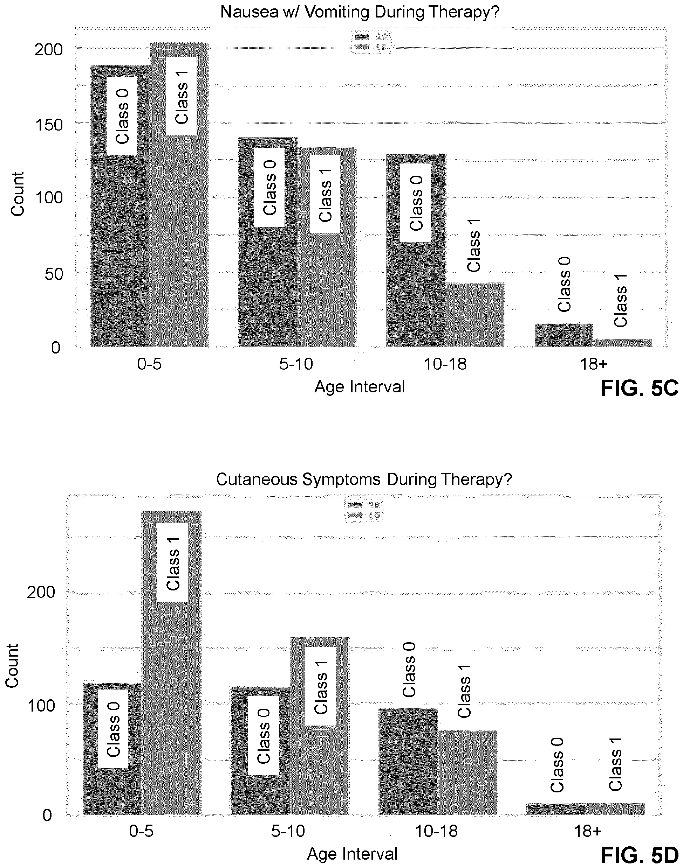

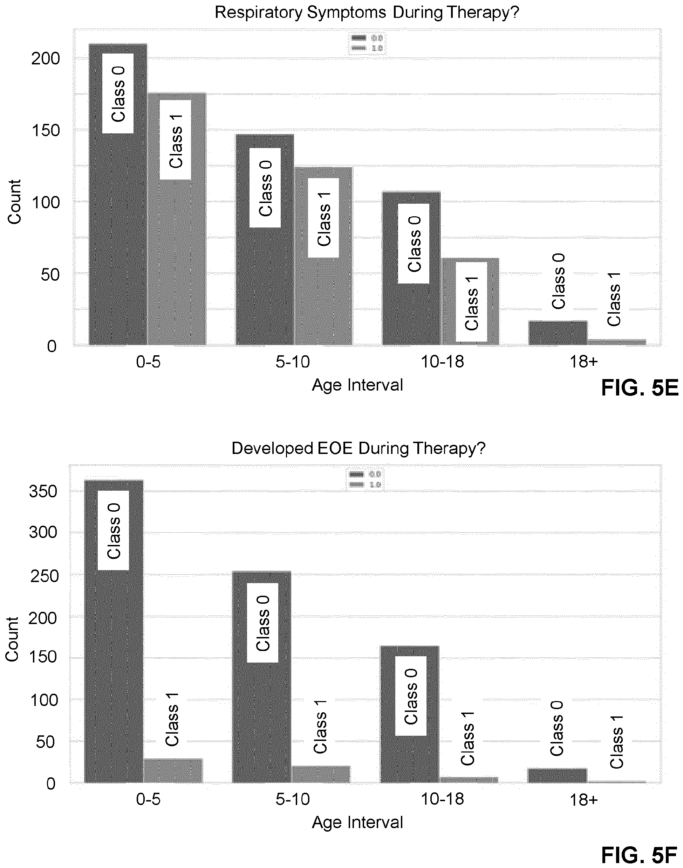

**FIGS. 5A-5F** illustrates a series of charts, each of which are directed to different symptoms. The count (y-axis) of patients in each of the age intervals (x-axis) experiencing or not experiencing the symptom is presented. Those who did not experience symptoms is represented by the bar to the left in each cluster. Those who did experience symptoms is represented by the bar to the right in each cluster.

It was determined that the most significant feature variables for symptom prediction include whether a patient is undergoing OIT for a single or multiple allergens, allergen specific serum, and age of the patient at the start of therapy.

Based on the foregoing, the present teachings propose building computer-based machine learning models to predict whether a patient will present a symptom and what symptom will be presented.

### Example 2.

The first part of modelling is determining whether patients will have symptoms or not.

Prediction parameters were collected for each patient and employed in the development of a machine learning model. Symptom features predicted via the ML model may be compared to actual symptoms (ground truths) to characterize the accuracy of the present method.

The ML model framework employed is known as the Light Gradient Boosting Method (LightGBM), although other suitable frameworks are contemplated by the present teachings.

Moreover, the present teachings contemplate employing a neural network for predicting whether patients will have symptoms or not.

80% of the data was used for training and 20% of the data was used to evaluate the performance of the machine learning model. The algorithm was trained and then an F1 score and an AUC score were employed to evaluate performance of the model.

Two classes were defined, those who did not actually present symptoms (Class 0) and those who actually presented symptoms (Class 1). The F1 score of classes 0 and 1 were 0.69 and 0.8, respectively. The AUC score was 88%. The performance evaluation indicated good overall accuracy of the model.

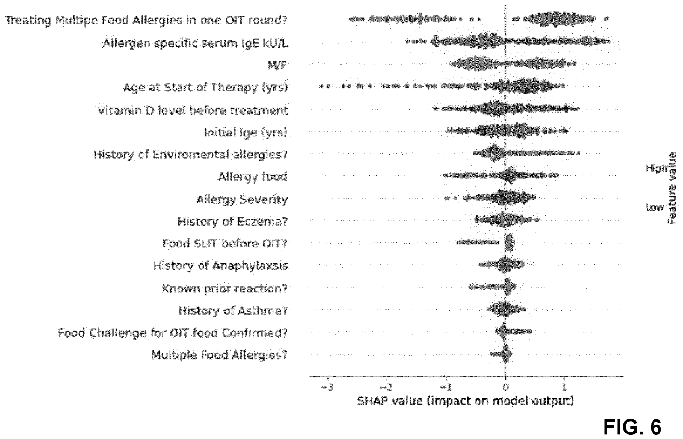

Next, the impact of each feature variable on the target variable was investigated. In this regard Shapley Additive explanations (SHAP) was employed. **FIG. 6** illustrates a graphical representation of the SHAP analysis. Dots on the right side of the line corresponds to those patients who experienced symptoms and dots on the left side of the line corresponds to those patients who did not experience symptoms. The results cooperate with the conclusion in **Example 1** that the most significant feature variables for symptom prediction include whether a patient is undergoing OIT for a single or multiple allergens, allergen specific Immunoglobulin E serum, and age of the patient at the start of therapy.

### Example 3.

The second part of modelling is which specific symptoms will be presented by the patients.

6 different ML models for each symptom were generated by using the LightGBM framework, although other suitable frameworks are contemplated by the present teachings. Moreover, the present teachings contemplate employing a neural network for predicting the types of symptoms the patient will present. Performance again was evaluated with an F1 score and an AUC score, provided below.

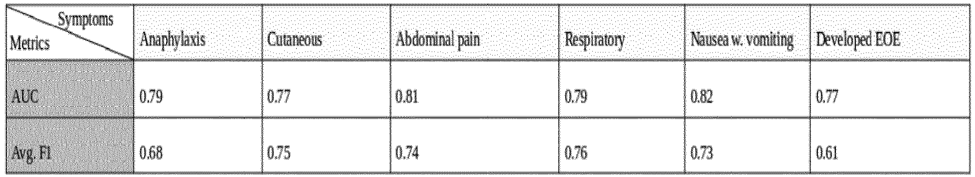

Anaphylaxis and eosinophilic esophagitis ("EOE") show comparatively lesser accuracy relative to the other symptoms due to the quantity of sampled patients who presented these symptoms.

### Example 4.

In some regards, data may be too sparse to obtain an accurate model. As discussed herein, training data sets may be dynamically updated, but this may not cure the challenge of sparse data. For example, a rare symptom presented in a patient with a rare food allergy may not be realized in an appreciable population size for some time, maybe even years. Moreover, new allergens and symptoms may arise over time, for which data will need to be collected.

In order to cure a sparse data set. Synthetic data may be generated. Thus, real data in a training set may be supplemented with synthetic data.

9257 synthetic records were constructed, with some data associated with Class 0 (experiencing no symptoms during OIT) and some data associated with Class 1 (experiencing symptoms with OIT). For each class, rows were replicated several times with the same values on several columns. The value of the numerical features including age at the start of OIT, skin prick test, and allergen specific Immunoglobulin E serum were changed by choosing a random number from the distribution of those numerical features. For the sake of simplicity, it was assumed that the features are normally distributed. In the end, the 9257 synthetic records were generated. All of the synthetic data was used for training and the original, genuine data set was used to test the accuracy of the model.

A single model was created for all symptoms. Thus, generating a multi-output classifier eliminates the burden of creating 6 different models for each symptom, decreasing the computational cost. One classifier per target were fit with the same hyper-parameters.

The LightGBM framework was used to this end, although other suitable frameworks are contemplated by the present teachings. Higher F1 and AUC scores were achieved with the synthetic data set compared to using the original, genuine data set alone. F1 and AUC scores are indicated below.

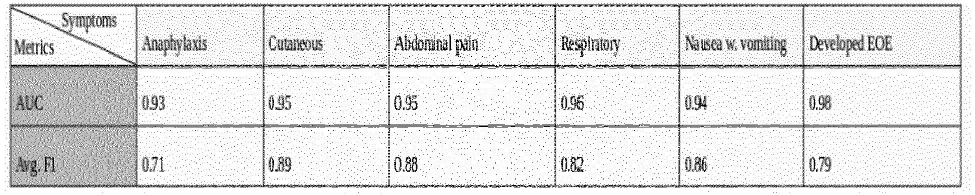

The columns and data structure of the models discussed herein are provided below.

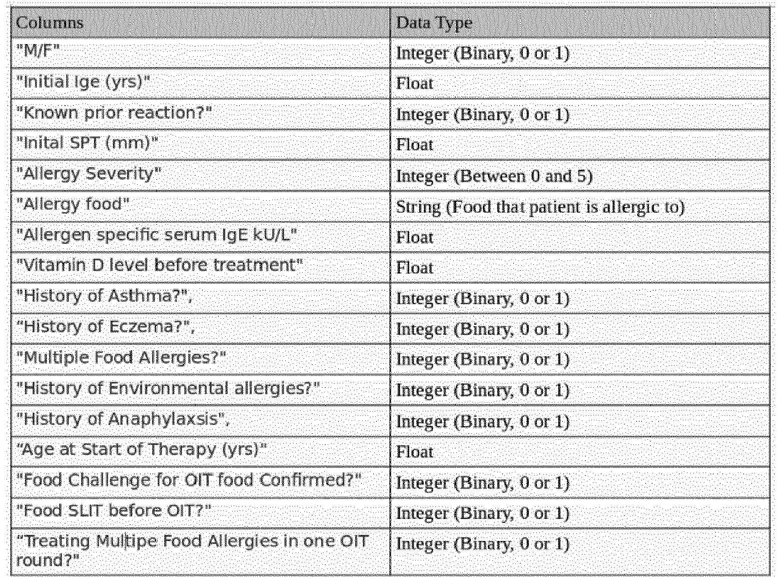

### Example 5.

A study was conducted on a population size of 866 patients, 60% of which were male, 82% of which had multiple food allergies, and 67% of which had environmental allergies. Prediction parameters discussed herein were tracked. There were a significant number of null values for vitamin D level, initial skin prick test, and immunoglobulin E.

The data was analyzed and the following observations were made.

It has been found that some prediction parameters provide a stronger correlation to the development of symptoms and/or the identity of those symptoms. These prediction parameters include allergen specific serum, age at the start of therapy, and whether a single food allergy or multiple food allergies are being treated in one round of immunotherapy. The trend for allergen specific serum was consistent among all symptoms. Generally, as allergen specific serum increases, so does the likelihood of having a symptom.

It has been found that more common symptoms during immunotherapy include cutaneous symptoms and abdominal pain. It has been found that the least common symptoms during immunotherapy include anaphylaxis and EOE.

It has been found that anaphylaxis, while occurring rarely, is more common amongst those being treated for multiple food allergies in one immunotherapy round. Cutaneous symptoms were exhibited by about 50% of those being treated for a single food allergy in one immunotherapy round and about 66% of those being treated for a single food allergy in one immunotherapy round. Abdominal pain and nausea were exhibited by about 30% of those being treated for a single food allergy in one immunotherapy round and about 50% of those being treated for multiple food allergies in one immunotherapy round.

It has been found that the instance of cutaneous symptoms decrease as age increases. It has been found that the instance of nausea with vomiting decreases as age increases. It has been found that as age increases the instance of abdominal pain increases.

### Example 6.

A model according to the present teachings was constructed. A population of data was obtained, which was split at random into a training set (80% of population) and a test set (20% of population). After training, the model was characterized with the test set for discrete symptoms.

The results are summarized below. Class 0 refers to those not showing symptoms. Class 1 refers to those showing symptoms. Scores can be represented as percentages. AUC score refers to the area under the ROC (receiver operating characteristic curve) having the false positive rate on the x-axis and true positive rate on the y-axis.

| Symptom exhibited during therapy | Class 0 F1 Score | Class 1 F1 Score | Avg. F1 Score | AUC Score | Patients in Class 0 / Classified Correctly | Patients in Class 1 / Classified Correctly |
|---|---|---|---|---|---|---|
| Anaphylaxis | 0.93 | 0.42 | 0.64 | 0.79 | 149/144 | 25/8 |
| Cutaneous | 0.68 | 0.81 | 0.76 | 0.74 | 67/44 | 107/88 |
| Abdominal pain and/or nausea without vomiting | 0.70 | 0.78 | 0.75 | 0.81 | 78/51 | 96/79 |
| Respiratory | 0.78 | 0.75 | 0.77 | 0.79 | 110/86 | 64/48 |
| Nausea with vomiting | 0.76 | 0.71 | 0.74 | 0.82 | 90/72 | 84/56 |
| EoE | 0.96 | 0.25 | 0.57 | 0.77 | 160/160 | 14/2 |

The foregoing examples first validate that correlations can be made between at least some prediction parameters and symptoms and then validate that said correlations can be detected with good accuracy in a ML model described herein.

As discussed in the several examples herein, some data may not be readily available due to the prevalence (or lack thereof) of patients being allergic to certain types of foods and experiencing certain symptoms. It is one aspect of the present disclosure that training data sets for the machine learning model and/or neural network may be dynamic. That is, the training data sets may be continuously updated and added to, and the machine learning model and/or neural network may continue to be improved thereby.

While the present disclosure discusses food as allergens, it is contemplated that the present teachings may be applied to any allergens, whether environmental (e.g., pollen), chemical (e.g., smoke), or the like.

The explanations and illustrations presented herein are intended to acquaint others skilled in the art with the invention, its principles, and its practical application. The above description is intended to be illustrative and not restrictive. Those skilled in the art may adapt and apply the invention in its numerous forms, as may be best suited to the requirements of a particular use. Other combinations are also possible as will be gleaned from the following claims, which are also hereby incorporated by reference into this written description.

Accordingly, the specific embodiments of the present invention as set forth are not intended as being exhaustive or limiting of the teachings. The scope of the teachings should, therefore, be determined not with reference to this description, but should instead be determined with reference to the appended claims, along with the full scope of equivalents to which such claims are entitled. The omission in the following claims of any aspect of subject matter that is disclosed herein is not a disclaimer of such subject matter, nor should it be regarded that the inventors did not consider such subject matter to be part of the disclosed inventive subject matter.

The disclosures of all articles and references, including patent applications and publications, are incorporated by reference for all purposes.

Plural elements or steps can be provided by a single integrated element or step. Alternatively, a single element or step might be divided into separate plural elements or steps.

The disclosure of "a" or "one" to describe an element or step is not intended to foreclose additional elements or steps.

While the terms first, second, third, etc., may be used herein to describe various elements, components, regions, layers, and/or sections, these elements, components, regions, layers, and/or sections should not be limited by these terms. These terms may be used to distinguish one element, component, region, layer, and/or section from another element, component, region, layer, and/or section. Terms such as "first," "second," and other numerical terms when used herein do not imply a sequence or order unless clearly indicated by the context. Thus, a first element, component, region, layer, and/or section discussed below could be termed a second element, component, region, layer, and/or section without departing from the teachings.

The use of "about" or "approximately" in connection with a range applies to both ends of the range. Thus, "about 20 to 30" is intended to cover "about 20 to about 30", inclusive of at least the specified endpoints.

Unless otherwise stated, any numerical values recited herein include both endpoints and all values from the lower value to the upper value in increments of one unit provided that there is a separation of at least 2 units between any lower value and any higher value. As an example, if it is stated that the amount of a component, a property, or a value of a process variable such as, for example, temperature, time, and the like is, for example, from 1 to 90, from 20 to 80, or from 30 to 70, it is intended that intermediate range values such as (e.g., 15 to 85, 22 to 68, 43 to 51, 30 to 32, etc.) are within the teachings of this specification. Likewise, individual intermediate values are also within the present teachings. For values which are less than one, one unit is considered to be 0.0001, 0.001, 0.01, or 0.1 as appropriate. These are only examples of what is specifically intended and all possible combinations of numerical values between the lowest value and the highest value enumerated are to be considered to be expressly stated in this application in a similar manner.

The terms "generally" or "about" to describe numbers or numerical ranges may mean ±0.2 for numbers from 0.1 to 1, ±2 for numbers from 2 to 100, and ±20 for numbers greater than 100. The foregoing is applicable to all percentages, temperatures, times, surface power densities, or otherwise, unless otherwise stated herein.

The term "consisting essentially of' to describe a combination shall include the elements, components, or steps identified, and such other elements, components, or steps that do not materially affect the basic and novel characteristics of the combination. The use of the terms "comprising" or "including" to describe combinations of elements, components, or steps herein also contemplates embodiments that consist essentially of the elements, components, or steps.

## Claims

**1.** A method for predicting an adverse symptom to immunotherapy, the method comprising:
receiving a medical history and one or more therapy features, related to a food allergy, of a patient;
evaluating with a machine learning model a probability of the patient experiencing, respectively, each of one or more symptoms during immunotherapy;
communicating the probability for each of the one or more symptoms to the patient and/or a physician of the patient.

**2.** The method according to Claim 1, wherein the medical history includes gender, one or more allergy foods, one or more allergy severities associated with the one or more allergy foods, one or more known prior reactions including a quantity and/or one or more symptom identifications, skin prick test results, allergen specific Immunoglobulin E antibody test results, allergen specific Immunoglobulin E antibody serum test results, vitamin D level, history of asthma, history of eczema, history of anaphylaxis, history of environmental allergies, history of environmental sublingual immunotherapy prior to oral immunotherapy, start and/or end date of the environmental sublingual immunotherapy, history of environmental subcutaneous immunotherapy prior to oral immunotherapy, start and/or end date of the environmental subcutaneous immunotherapy, epinephrine autoinjector use history during the environmental sublingual immunotherapy, epinephrine autoinjector use history during the environmental subcutaneous immunotherapy, or any combination thereof; preferably wherein the medical history includes at least allergen specific Immunoglobulin E antibody serum test results.

**3.** The method according to Claim 1 or Claim 2, wherein the one or more therapy features includes age at the start of immunotherapy, food challenge history prior to oral immunotherapy, food sublingual immunotherapy history prior to oral immunotherapy, whether the oral immunotherapy involves treatment for a single or multiple food allergies, or any combination thereof; preferably wherein the one or more therapy features includes at least age at the start of immunotherapy and whether the oral immunotherapy involves treatment for a single or multiple food allergies.

**4.** The method according to any one of the preceding claims, wherein the immunotherapy is oral immunotherapy in which the patient orally ingests a medication comprising a component of an allergen food on a predetermined schedule, including a plurality of maintenance phases in which a dosage remains constant and a plurality of up-dosing phases in which the dosage is increased relative to an immediately prior maintenance phase.

**5.** The method according to any one of the preceding claims, wherein the one or more symptoms include anaphylaxis, cutaneous symptoms, respiratory symptoms, abdominal pain and/or nausea without vomiting, nausea with vomiting, development of eosinophilic esophagitis, or any combination thereof.

**6.** The method according to any one of the preceding claims, wherein the machine learning model includes a light gradient-boosting machine framework; and optionally wherein the machine learning model includes a logistic regression algorithm.

**7.** The method according to any one of the preceding claims, wherein the machine learning model is trained by supervised learning.

**8.** The method according to any one of the preceding claims, wherein the machine learning model is trained with one or more training sets of data comprising genuine patient data, synthetic data, or both; optionally wherein the genuine patient data is anonymized.

**9.** The method according to any one of the preceding claims, wherein the one or more training sets of data undergo one or more transformations including replacing missing values, encoding categorical data into numerical data, standardizing data scales, balancing, or any combination thereof; optionally wherein the balancing excludes at least some of the one or more training sets of data such that a ratio of data sets where no symptoms are experienced during therapy to data sets where symptoms are experienced during therapy is about 70:30 or less, more preferably about 65:35 or less, more preferably 60:40 or less, more preferably about 55:45 or less, or even more preferably about 50:50.

**10.** The method according to any one of the preceding claims, wherein the medical history and the one or more therapy features are received by a computing device of the patient, the physician, or both; optionally wherein the medical history and the one or more therapy features each include a first component and a second component, whereby the first component is received from the patient and the second component is received from the physician.

**12.** A non-transient memory storage medium comprising computer executable instructions for performing a method according to any one of the preceding claims.

**13.** The non-transient memory storage medium according to Claim 12, wherein the non-transient memory storage medium is local to a computing device of the patient or local to a computing device of the physician.

**14.** The non-transient memory storage medium according to Claim 12 or Claim 13, wherein the computer executable instructions are carried out by one or more processors local to the computing device of the patient or local to the computing device of the physician.
